# EUROPÄISCHE PATENTSCHRIFT

(11) **EP 0 901 785 B1**
(45) Veröffentlichungstag und Bekanntmachung des Hinweises auf die Patenterteilung: **22.11.2006**
(21) Anmeldenummer: 98117258.8
(22) Anmeldetag: 11.09.1998
(51) Int. Cl.: A61K 6/10

(54) **Dentalmassen auf Polyetherbasis**
Polyether based dental compositions
Compositions dentaires à base de polyéthers

(30) Priorität: 12.09.1997 DE 19740234
(43) Veröffentlichungstag der Anmeldung: 17.03.1999
(73) Patentinhaber: 3M ESPE AG, 82229 Seefeld (DE)
(72) Erfinder: Eckhardt, Gunther, 82346 Frieding (DE); Wanek, Erich, 86916 Kaufering (DE); Lechner, Günter, 82237 Wörthsee (DE); Bissinger, Peter, 84615 Mering (DE); Mikulla, Markus, 82346 Andechs-Frieding (DE)

(56) Entgegenhaltungen:
- EP-A- 0 153 794
- EP-A- 0 421 371
- DE-A- 1 745 810
- DE-A- 3 246 654
- DE-A- 3 514 547
- DE-A- 3 607 946

## Beschreibung

Die Erfindung betrifft dentale Abformmassen auf der Basis von Polyetherderivaten, ihre Herstellung und ihre Verwendung Abformung im Zahnmedizinischen oder Zahntechnischen Bereich.

Die Herstellung von Polyetherderivaten und ihre Verwendung in Dentalmaterialien ist seit langem bekannt. So beschreibt beispielsweise die DE-C-1 745 810 die Herstellung von Formkörpern auf der Basis von Aziridino-Polyethern.

In den Patentschriften DE-C-3 246 654, EP-A-0 421 371 und EP-A-0 110 429 ist die Verwendung von Aziridino-Polyethern in Polyetherabformmassen beschrieben. Lichthärtende Abformmaterialien auf der Grundlage von Polyether-Urethanacrylaten beschreibt die EP-A-0 460 478.

Die Abformung der konkreten Verhältnisse im Mund des Patienten mit Hilfe geeigneter Abformmassen ist die Voraussetzung zur Herstellung von paßgenauen Prothesen, Kronen und Brücken, Inlays und Onlays.

Von den bekannten Abformmassen zeichnen sich die auf Polyetherderivaten basierenden Massen durch ihren hydrophilen Charakter aus, was eine sehr hohe Präzision der Abdrücke möglich macht.

Nachteilig ist an diesen Massen allerdings, daß sie sich nicht allzu leicht entformen lassen. Das heißt, daß die Entformbarkeit des Abdrucks bei der Abdrucknahme sowie die Entformbarkeit des Gipsmodells nach dem Ausgießen des Abdrucks nicht zufriedenstellend sind.

Aufgabe der vorliegenden Erfindung ist es, dentale Abformmassen auf der Basis von Polyetherderivaten bereitzustellen, die die geschilderten Nachteile nicht aufweisen, sondern leicht entformbar sind.

Gelöst wird die Aufgabe durch dentale Abformmassen auf der Basis von Polyetherderivaten, die dadurch gekennzeichnet sind, daß ihr Gehalt an cyclischen oligomeren Polyethern kleiner als 0,9 Gew.-% ist.

Überraschenderweise wurde im Rahmen der vorliegenden Erfindung festgestellt, daß die in den Polyethermassen vorhandenen cyclischen Polyetheroligomeren dafür verantwortlich sind, daß die Massen eine schlechte Entformbarkeit des Abdrucks bei der Abdrucknahme sowie eine schlechte Entformbarkeit des Gipsmodelles nach dem Ausgießen des Abdrucks aufweisen.

Die Grundlage für die in Dentalmaterialien eingesetzten Polyetherderivate stellen Polyetherpolyole dar, die nach unterschiedlichen Polymerisationsverfahren hergestellt werden können und in der Regel Molmassen im Bereich von 500 bis 10.000 g/Mol aufweisen.

Es ist bereits bekannt (G. Pruckmayr et al., ACS Symp. Ser. 172, (1981), S. 197 bis 203), daß bei der Herstellung von Polyetherglykolen durch Homopolymerisation von Tetrahydrofuran oder Ethylenoxid bzw. durch Copolymerisation von Tetrahydrofuran mit Ethylenoxid unter katalytischer Wirkung starker Säuren cyclische Oligomere neben linearen Polyetherglykolen entstehen und daß der Gehalt solcher cyclischer Oligomerer in Abhängigkeit von den Reaktionsbedingungen bis zu 20 Gew.-% betragen kann (DE-A-3 514 547) und üblicherweise bei technischen Polyetherpolyolen unter 10 Gew.-% liegt.

Diese cyclischen Oligomeren besitzen in Abhängigkeit von der Comonomerzusammensetzung und den Reaktionsbedingungen unterschiedliche Ringgrößen und bzw. oder unterschiedliche Verhältnisse der eingebauten Monomereinheiten.

Im Stand der Technik sind weiterhin Verfahren beschrieben, die die Entfernung der cyclischen Oligomeren betreffen. So beschreibt die EP-A-0 153 794 die Entfernung der cyclischen Oligomeren durch Extraktion mittels Heptan.

Extraktionsverfahren zur Entfernung der cyclischen Oligomeren sind auch in den DE-A-3 514 547, DE-A-3 607 946 und DE-A-3 730 888 beschrieben.

Gemäß EP-A-0 305 853 wird eine Dreiphasen-Extraktion mit vorgeschalteter Vakuumdestillation in einem Kurzwegverdampfer vorgeschlagen.

In der DE-A-195 30 388 werden geruchsarme, höhermolekulare Polyetherpolyole beschrieben, die durch Zugabe von Wasser bei Temperaturen von 110 bis 150°C und unter vermindertem Druck gereinigt werden. Dieser Reinigungsprozeß führt zu einer Verringerung des Gehaltes an geruchsintensiven Verbindungen und macht die so gereinigten Polyether geeignet für die Herstellung von auf Polyetherpolyolen aufbauenden emissionsarmen Polymeren, Kosmetika und pharmazeutischen Produkten.

Bei dentalen Abformmassen ist die Entfernung der cyclischen Oligomeren bislang nicht in Betracht gezogen worden, da diese Dentalmassen ausgezeichnete Eigenschaften aufweisen und somit kein Bedarf bestand, die cyclischen Oligomeren zu entfernen. Überraschenderweise wurde im Rahmen der Erfindung, wie schon oben erwähnt, festgestellt, daß die schwierige Entformbarkeit der Polyethermassen auf die Anwesenheit der cyclischen Oligomeren zurückzuführen ist. Durch ihre Entfernung werden dentale Abformmassen auf Polyetherbasis erhalten, die eine unverändert gute Abdruckpräzision besitzen, gleichzeitig aber auch leicht entformbar sind.

Bei vorgegebenem Einbau-Verhältnis der Monomeren kann der Gesamtgehalt der cyclischen oligomeren Polyether, die Verteilung der einzelnen Typen untereinander und damit die Molmassenverteilung der cyclischen Oligomeren durch die Reaktionstemperatur und den realisierten Konzentrationsverlauf der Monomeren über die Reaktionszeit beeinflußt werden.

Die analytische Bestimmung des Gehaltes an cyclischen oligomeren Polyethern und der Verteilung der einzelnen Oligomertypen kann gaschromatographisch mit FID-Detektor bzw. in GC-MS-Kopplung realisiert werden.

Die Entfernung der cyclischen oligomeren Polyether kann sowohl auf der Verfahrensstufe der Polyetherpolyole als auch nach deren Funktionalisierung mit Aziridino-Gruppen, Doppelbindungs-haltigen Gruppen und Epoxidgruppen erfolgen, wobei destillative und extraktive Verfahren oder die Membrantrennung anwendbar sind.

Die destillativen Trennverfahren sind mit der Anwendung von hohen Temperaturen und damit den Gefahren der thermischen Schädigung verbunden. So können die destillativ aufgereinigten Polyetherpolyole einen meist unangenehmen Geruch aufweisen.

Die destillative Reinigung der bereits funktionalisierten Polyetherpolyole ist wegen der Gefahr der vorzeitigen Polymerisation schwierig durchzuführen. Für die extraktive Entfernung der cyclischen oligomeren Polyether ist die Extraktion mit Kohlenwasserstoffen mit 4 bis 12 C-Atomen prinzipiell geeignet. Die Extraktion kann kontinuierlich oder diskontinuierlich gemäß den bekannten Flüssig/Flüssig-Separationsverfahren (s. Ullmann's Encyclopedia of Industrial Chemistry, 5. Ausgabe, Volume B3: Unit Operations) durchgeführt werden. Die extraktive Entfernung der cyclischen oligomeren Polyether mittels Kohlenwasserstoffen ist sowohl auf der Stufe der Polyetherpolyole als auch im Anschluß an die Funktionalisierung möglich. Es hat sich bei der Herstellung von Polyetherderivaten für den Einsatz in Dentalmaterialien als vorteilhaft erwiesen, die Reinigungsstufe der Cyclenentfernung durch Extraktion mit den Reinigungsstufen nach der Funktionalisierung zu kombinieren.

Die für die Funktionalisierung eingesetzten Polyetherpolyole werden bevorzugt durch Copolymerisation von Tetrahydrofuran und Ethylenoxid im Molverhältnis 10 : 1 bis 1: 1 und vorzugsweise 5 : 1 bis 3 : 1 in Gegenwart starker Säure, wie beispielsweise Borfluorid-Etheraten hergestellt.

Es ist ebenfalls möglich, für die Funktionalisierung Polyetherpolyole einzusetzen, die neben Tetrahydrofuran-Einheiten auch Ethylenoxid-Einheiten und bzw. oder Propylenoxid-Einheiten enthalten.

Die Polyetherpolyole besitzen mindestens 2 Hydroxylgruppen, können aber auch bis zu 20 Hydroxylgruppen pro Molekül enthalten.

Die Molmassen (Mₙ) der zur Funktionalisierung eingesetzten Polyetherpolyole liegen im Bereich von 500 bis 20.000 und bevorzugt im Bereich von 2.000 bis 10.000 g/Mol. Die Funktionalisierung mit Aziridinogruppen kann beispielsweise nach dem in der DE-C-1 745 810 beschriebenen Verfahren erfolgen.

Eine Funktionalisierung mit Epoxidgruppen, wie beispielsweise 3,4-Epoxycyclohexylgruppen ist nach der Lehre der DE-A-195 34 668 möglich. Die Funktionalisierung mit (Meth-)acrylatgruppen kann beispielsweise gemäß der DE-A-4 406 858, Beispiel 1, erfolgen. Für die Funktionalisierung mit Allylgruppen, Vinylgruppen, Vinylethergruppen und Maleatgruppen sind die bekannten Verfahren der Umsetzung primärer Alkohole anwendbar.

Aus den funktionalisierten Polyetherderivaten können dentale Abformmassen gefertigt werden, die durch polymerbildende Reaktionen aushärten. Bevorzugte polymerbildende Reaktionen sind die radikalische und die kationische Polymerisation sowie die Hydrosilylierung.

Der Einsatz der weitgehend von cyclischen oligomeren Polyethern befreiten, funktionalisierten Polyetherderivate kann in sehr unterschiedlichen zahnmedizinisch oder zahntechnisch verwendeten Dentalmassen erfolgen. Bevorzugte Einsatzgebiete sind die einphasige und die zweiphasige zahnmedizinische Abformung und die Bißregistrierung.

Die Erfindung wird anhand der folgenden Beispiele weiter erläutert.

### Beispiele

Die Herstellung eines Mischpolyether-Dimethacrylates mit einem niedrigen Gehalt an cyclischen Oligomeren erfolgte gemäß Herstellungsbeispiel 1. Das Herstellungsbeispiel 2 beschreibt die Herstellung eines Bis-Aziridinopolyethers mit einem niedrigen Gehalt an cyclischen oligomeren Polyethern, ausgehend von einem Bis-Aziridinopolyether, der gemäß der DE-C-1 745 810 erhalten wurde.

Die Bestimmung des Restgehalts der cyclischen oligomeren Polyether in den Polyetherderivaten der Herstellungsbeispiele 1 und 2 erfolgte gaschromatographisch.

### Analysenmethode

Für die gaschromatographischen Messungen wurde ein Gaschromatograph (CP 9000) mit FID-Detektor der Firma Chrompack verwendet. Die Temperatur des Detektorblocks betrug 330°C. Als Säule wurde eine 10 m DMS-Kapillare der Firma Chrompack verwendet. Wasserstoff diente als Trägergas, der Trägergasdruck betrug 30 kPa. Die Trennungen wurden temperaturprogrammiert (Start 100°C, Ende 300°C, Anstiegsrate 20°C/min) durchgeführt. Es wurde jeweils 1 Mikroliter der Prüflösungen (2 Gew.-% in Dichlormethan) eingespritzt. Als interner Standard wurde der Kronenether 15-Krone-5 (Fa. Merck) zugesetzt. Die Peakflächen wurden mit Hilfe der Auswerte-Software Turbochrom (Fa. PE-Nelson) berechnet.

Die Polyetherderivate der Herstellungsbeispiele 1 und 2 wurden zur Herstellung der Dentalmaterialien der Beispiele 1 bis 7 verwendet.

Die Entnehmbarkeit des Abdrucks wurde jeweils an 8 Probanden mit unterschiedlichen Gebißsituationen von 2 Durchführenden bewertet und die subjektiven Eindrücke gemittelt.

Es wurde folgendes Bewertungsschema für die Entnehmbarkeit zugrunde gelegt:
- 1 =: sehr gut
- 2 =: gut
- 3 =: genügend
- 4 =: mangelhaft
- 5 =: schlecht

### Herstellungsbeispiel 1

### Herstellung eines Mischpolyether-Dimethacrylates mit niedrigem Gehalt an cyclischen Oligomeren

Ein Mischpolyetherdiol mit einer Molmasse (Mₙ) von 6500, hergestellt durch kationische Copolymerisation von Ethylenoxid und Tetrahydrofuran im Molverhältnis 1 : 3 wurde durch Umsetzung mit Methacrylsäureanhydrid unter katalytischer Wirkung von Kaliumhydroxid analog der in der DE-A-4 406 858, Beispiel 1 beschriebenen Verfahrensweise zum Mischpolyether-Dimethacrylat umgesetzt.

Die Reaktionsmischung wurde unter Rühren durch Zugabe einer 2 %-igen wäßrigen Lösung von Kaliumhydroxid neutral gestellt. Die zweiphasige Mischung wurde mit Heptan überschichtet und die dreiphasige Mischung bei 10°C 2 Stunden lang gerührt. Nach Entfernung der Haptanphase erfolgte eine erneute Überschichtung mit Heptan. Dieser Vorgang wurde 3x wiederholt und anschließend wurden die Heptanphase sowie die wäßrige Phase abgetrennt.

Aus der mittleren Phase wurde nach Zugabe von 200 ppm 4-Methoxyphenol durch Vakuumdestillation bei 50°C das restliche Wasser entfernt.

Das erhaltene Mischpolyether-Dimethacrylat war farblos und hatte eine Doppelbindungs-Äquivalentmasse von 3420 g/Mol.

Der Gehalt an cyclischen Polyetheroligomeren lag, nach der beschriebenen gaschromatographischen Methode bestimmt, bei 0,09 Gew.-%.

### Herstellungsbeispiel 2

### Herstellung eines cyclenfreien Aziridino-Polyethers

500 Gewichtsteile eines Aziridino-Polyethers, hergestellt gemäß der Lehre der DE-C-1 745 810 mit eine zahlenmittleren Molmasse von 6100 g/Mol und einem Einbauverhältnis von Ethylenoxid- zu Tetrahydrofuran-Einheiten von 1 : 3,6, enthaltend 8,2 Gewichtsteile an cyclischen oligomeren Polyethern, der nach der Funktionalisierung mit Aziridinogruppen 5x mit Wasser gewaschen worden war, wurde ohne Zwischentrocknung mit 300 Gewichtsteilen Hexan überschichtet und bei 20°C gerührt. Die obere Phase (Hexanphase) wurde entfernt und der Rückstand erneut mit 300 Gewichtsteilen Hexan überschichtet.

Dieser Vorgang wurde 7x wiederholt und anschließend wurden die Hexanphase sowie die wäßrige Phase abgetrennt. Nach Aufarbeitung der Hexanphase wurden 450 Gewichtsteile eines Bis-Aziridino-Polyethers mit einem Gehalt von 0,25 Gew.-% an cyclischen oligomeren Polyethern erhalten.

### Herstellungsbeispiele 3 bis 5 und Herstellungs-Vergleichsbeispiel 1 Herstellung der Basiskomponenten

Durch Vermischen der nachfolgend charakterisierten Zubereitungen wurden jeweils 100 Gewichtsteile der Basiskomponenten erhalten.

| Bestandteile | Herstellungsbeispiel 3 Basispaste B1 Gew.-% | Herstellungsbeispiel 4 Basispaste B2 Gew.-% | Herstellungsbeispiel 5 Basispaste B3 Gew.-% | Herstellungsvergleichsbeispiel 1 Basispaste VB 1 Gew.-% |
|---|---|---|---|---|
| -Azirdino-Polyether gemäß Herstellungsbeispiel 2 | 57,1 | 53,8 | 51,3 | - |
| -Dibenzyltoluol | 12,4 | 13,9 | 16,3 | 11,2 |
| -Hydrierter Rindertalg | 14,6 | 15,8 | 15,1 | 14,5 |
| -Kieselgur (Diatomeenerde) | 13,3 | 13,9 | 14,7 | 13,9 |
| -Farbpigment | 2,6 | 2,6 | 2,6 | 2,6 |
| -Aziridino-Polyether, hergestellt gemäß DE-C-1 745 810, Mₙ = 6100 g/Mol Einbauverhältnis von Ethylenoxid- zu Tetrahydrofuran-Einheiten von 1:3,6; enthaltend 8,2 Gew.-% an cyclischen Oligomeren | - | - | - | 57,8 |

### Herstellungsbeispiel 6

### Herstellung der Katalysatorkomponente K 1

32,9 Gewichtsteile eines Sulfoniumsalzes, das gemäß Beispiel 27 der DE-A-2 515 593 erhalten wurde, 32,0 Gewichtsteile Acetyltributylcitrat, 5,8 Gewichtsteile eines Block-Copolymer-Surfactants aus Propylenoxid und Ethylenoxid mit einer mittleren Molmasse von 6500, 19,1 Gewichtsteile pyrogene Kieselsäure, 9,5 Gewichtsteile Kieselgur und 0,7 Gewichtsteile Farbpigmente wurden zu 100 Gewichtsteilen der Katalysatorpaste K 1, die zur Aushärtung von Basiskomponenten eingesetzt wird, verknetet.

### Beispiele 1 bis 3 sowie Vergleichsbeispiel 1

### Herstellung von Abdrücken

Die Katalysatorkomponenten und die Basiskomponenten wurden, wie nachfolgend angegeben, im Gewichtsverhältnis von 1 : 5 auf dem Anmischblock gemischt, die Mischungen auf ein Metalltray übertragen und der gefüllte Abdrucklöffel in den Mund des Probanden eingeführt. Nach einer Mundverweildauer von 6 Minuten, gerechnet von Mischbeginn, wurden die Abdrücke entnommen.

### Zusammensetzung der im Verhältnis 1 : 5 gemischten Abformmassen

| | Katalysatorpaste | Basispaste |
|---|---|---|
| Beispiel 1 | K 1 | B 1 |
| | (gem. Herstellungsbeispiel 6) | (gem. Herstellungsbeispiel 3) |
| Beispiel 2 | K 1 | B 2 |
| | | (gem. Herstellungsbeispiel 4) |
| Beispiel 3 | K 1 | B 3 |
| | | (gem. Herstellungsbeispiel 5) |
| Vergleichs- | K 1 | VB 1 |
| beispiel 1 | | (gem. Herstellungsvergleichsbeispiel 1) |

Die Entnehmbarkeit der Abdrücke wurde in der beschriebenen Weise bewertet und ist in nachfolgender Tabelle angegeben.

### Ergebnisse der Ausprüfung der Massen gemäß den Beispielen 1 bis 3 und Vergleichsbeispiel 1

| | Durchschnittswert "Entnehmbarkeit" |
|---|---|
| Beispiel 1 | 2,7 |
| Beispiel 2 | 1,6 |
| Beispiel 3 | 1,3 |
| Vergleichsbeispiel 1 | 4,3 |

Der Vergleich der Ergebnisse der erfindungsgemäßen Beispiele mit denen des Vergleichsbeispiels 1 zeigt die Überlegenheit der erfindungsgemäßen Massen.

### Herstellungsbeispiel 7

### Herstellung einer Abformmasse

59,3 Gewichtsteile des Mischpolyether-Dimethacrylates gemäß Herstellungsbeispiel 1 wurden mit 0,9 Gewichtsteilen Lucirin TPO (BASF), 30 Gewichtsteilen eines Urethandimethacrylates mit einer Doppelbindungsäquivalentmasse von 245 g/Mol und 6,1 Gewichtsteilen eines Multiacrylates mit einer Molmasse von 880 g/Mol und einer Doppelbindungsäquivalentmasse von 250 g/Mol versetzt und die Mischung durch Zusatz von 3,7 Gewichtsteilen hochdispersiver Kieselsäure (HDK N20, Fa. Wacker) thixotropiert.

### Beispiel 4

Die in Herstellungsbeispiel 7 erhaltene Abformmasse wurde auf ein Kunststofftray, das eine hohe Transparenz besitzt, aufgetragen und der gefüllte Abdrucklöffel in den Mund des Probanden eingeführt.

Die Belichtung der Abformmasse erfolgte durch den Kunststoff des Abdrucklöffels mittels einer Lampe, die sichtbares Licht im Wellenlängenbereich von 400 bis 500 nm ausstrahlt.

Der Abdruck war nach 2 1/2 Minuten entnehmbar.

Die Prozedur wurde an 8 Probanden durchgeführt, die übereinstimmend die Abdrucknahme als nur wenig belastend beschrieben. Demgegenüber wird eine Abdrucknahme mit der Abformmasse des Vergleichsbeispiels 1 als unangenehm beschrieben.

## Patentansprüche

1. Dentale Abformmasse auf der Basis von Polyetherderivaten, **dadurch gekennzeichnet, daß** der Gehalt der cyclischen oligomeren Polyether in den Polyetherderivaten kleiner als 0,9 Gew.-% ist.

2. Dentale Abformmasse nach Anspruch 1, **dadurch gekennzeichnet, daß** der Gehalt der cyclischen oligomeren Polyether mit Molmassen unter 500 g/Mol in den Polyetherderivaten kleiner als 0,5 Gew.-% ist.

3. Dentale Abformmasse nach den Ansprüchen 1 oder 2, **dadurch gekennzeichnet, daß** der Gehalt der cyclischen oligomeren Polyether mit Molmassen unter 350 g/Mol in den Polyetherderivaten kleiner als 0,2 Gew.-% ist.

4. Dentale Abformmasse nach den Ansprüchen 1 bis 3, **dadurch gekennzeichnet, daß** sie aus einem Polyetherpolyol hergestellt worden ist, das seinerseits durch Copolymerisation von Tetrahydrofuran mit Ethylenoxid in Gegenwart starker Säuren erzeugt wurde.

5. Dentale Abformmasse nach den Ansprüchen 1 bis 3, **dadurch gekennzeichnet, daß** sie aus einem Polyetherpolyol hergestellt worden ist, das neben Tetrahydrofuran-Einheiten Ethylenoxid-Einheiten und/oder Propylenoxid-Einheiten enthält.

6. Dentale Abformmasse nach den Ansprüchen 1 bis 5, **dadurch gekennzeichnet, daß** die Polyetherderivate mit Aziridinogruppen funktionalisiert sind.

7. Dentale Abformmasse nach den Ansprüchen 1 bis 5, **dadurch gekennzeichnet, daß** die Polyetherderivate mit Doppelbindungs-haltigen Gruppen, wie Acrylgruppen, Methacrylgruppen, Allylgruppen, Vinylgruppen, Vinylethergruppen und Maleatgruppen funktionalisiert sind.

8. Dentale Abformmasse nach den Ansprüchen 1 bis 4, **dadurch gekennzeichnet, daß** die Polyetherderivate mit Epoxidgruppen funktionalisiert sind.

9. Verfahren zur Herstellung von dentalen Abformmassen nach den Ansprüchen 1 bis 8 enthaltend polyetherderivate, **dadurch gekennzeichnet, daß** die cyclischen oligomeren Polyether aus den Polyetherpolyolen vor der Funktionalisierung mit Aziridinogruppen und/oder Doppelbindungs-haltigen Gruppen und/oder Epoxidgruppen mittels Membrantrennung entfernt werden, so dass der Gehalt der cyclischen oligomeren Polyether in den Polyetherderivaten kleiner als 0,9 Gew.-% ist.

10. Verfahren zur Herstellung von dentalen Abformmassen nach den Ansprüchen 1 bis 8 enthaltend Polyetherderivate, **dadurch gekennzeichnet, daß** die cyclischen oligomeren Polyether nach der Funktionalisierung der Polyetherpolyole mit Aziridinogruppen und/oder Doppelbindungs-haltigen Gruppen und/oder Epoxidgruppen auf extraktivem Wege entfernt werden, so dass der Gehalt der cyclischen oligomeren Polyether in den Polyetherderivaten kleiner als 0,9 Gew.-% ist.

11. Verfahren zur Herstellung von dentalen Abformmassen nach den Ansprüchen 9 oder 10, **dadurch gekennzeichnet, daß** die Entfernung der cyclischen oligomeren Polyether durch eine Extraktion mit C₄- bis C₁₂-Kohlenwasserstoffen erfolgt.

12. Verwendung der dentalen Abformmasse nach den Ansprüchen 1 bis 8 zur Abformung im zahnmedizinischen oder zahntechnischen Bereich.

## Claims

1. Dental impression composition based on polyether derivatives, **characterized in that** the level of cyclic oligomeric polyethers in the polyether derivatives is less than 0.9 wt.-%.

2. Dental impression composition according to Claim 1, **characterized in that** the level of cyclic oligomeric polyethers having molar masses below 500 g/mol in the polyether derivatives is less than 0.5 wt.-%.

3. Dental impression composition according to Claims 1 or 2, **characterized in that** the level of cyclic oligomeric polyethers having molar masses below 350 g/mol in the polyether derivatives is less than 0.2 wt.-%.

4. Dental impression composition according to Claims 1 to 3, **characterized in that** they are produced from a polyether polyol which in turn was produced by copolymerization of tetrahydrofuran with ethylene oxide in the presence of strong acids.

5. Dental impression composition according to Claims 1 to 3, **characterized in that** they are produced from a polyether polyol which, as well as tetrahydrofuran units, contains ethylene oxide units and/or propylene oxide units.

6. Dental impression composition according to Claims 1 to 5, **characterized in that** the polyether derivatives are functionalized with aziridino groups.

7. Dental impression composition according to Claims 1 to 5, **characterized in that** the polyether derivatives are functionalized with double-bond-containing groups, such as acryloyl groups, methacryloyl groups, allyl groups, vinyl groups, vinyl ether groups and maleate groups.

8. Dental impression composition according to Claims 1 to 4, **characterized in that** the polyether derivatives are functionalized with epoxide groups.

9. Process for producing dental impression compositions according to Claims 1 to 8, containing polyether derivatives, **characterized in that** the cyclic oligomeric polyethers are removed from the polyether polyols by membrane separation before the functionalization with aziridino groups and/or double-bond-containing groups and/or epoxide groups such that the level of cyclic oligomeric polyethers in the polyether derivatives is less than 0.9% by weight.

10. Process for producing dental impression compositions according to Claims 1 to 8, containing polyether derivatives, **characterized in that** the cyclic oligomeric polyethers are removed extractively after the functionalization of the polyether polyols with aziridino groups and/or double-bond-containing groups and/or epoxide groups such that the level of cyclic oligomeric polyethers in the polyether derivatives is less than 0.9% by weight.

11. Process for producing dental impression compositions according to Claims 9 or 10, **characterized in that** the cyclic oligomeric polyethers are removed by an extraction with C₄- to C₁₂-hydrocarbons.

12. Use of the dental impression composition according to Claims 1 to 8 for impression taking in dental medicine or dental engineering.

## Revendications

1. Masse de moulage dentaire à base de dérivés de polyéther, **caractérisée en ce que** la teneur en polyéthers oligomères cycliques dans les dérivés de polyéther est inférieure à 0,9% en poids.

2. Masse de moulage dentaire selon la revendication 1, **caractérisée en ce que** la teneur en polyéthers oligomères cycliques présentant des masses molaires inférieures à 500 g/mole dans les dérivés de polyéther est inférieure à 0,5% en poids.

3. Masse de moulage dentaire selon les revendications 1 ou 2, **caractérisée en ce que** la teneur en polyéthers oligomères cycliques présentant des masses molaires inférieures à 350 g/mole dans les dérivés de polyéther est inférieure à 0,2% en poids.

4. Masse de moulage dentaire selon les revendications 1 à 3, **caractérisée en ce qu'**elle est préparée à partir d'un polyétherpolyol qui est produit à son tour par copolymérisation de tétrahydrofurane avec de l'oxyde d'éthylène en présence d'acides forts.

5. Masse de moulage dentaire selon les revendications 1 à 3, **caractérisée en ce qu'**elle est préparée à partir d'un polyétherpolyol, qui contient, outre des unités de tétrahydrofurane, des unités d'oxyde d'éthylène et/ou des unités d'oxyde de propylène.

6. Masse de moulage dentaire selon les revendications 1 à 5, **caractérisée en ce que** les dérivés de polyéther sont fonctionnalisés avec des groupes aziridino.

7. Masse de moulage dentaire selon les revendications 1 à 5, **caractérisée en ce que** les dérivés de polyéther sont fonctionnalisés avec des groupes contenant des doubles liaisons, tels que des groupes acryle, méthacryle, allyle, vinyle, vinyléther et maléate.

8. Masse de moulage dentaire selon les revendications 1 à 4, **caractérisée en ce que** les dérivés de polyéther sont fonctionnalisés avec des groupes époxyde.

9. Procédé pour la préparation de masses de moulage dentaires selon les revendications 1 à 8, contenant des dérivés de polyéther, **caractérisé en ce que** les polyéthers oligomères cycliques sont éliminés des polyétherpolyols avant la fonctionnalisation avec des groupes aziridino et/ou des groupes contenant des doubles liaisons et/ou des groupes époxyde au moyen d'une séparation sur membrane, de telle manière que la teneur en polyéthers oligomères cycliques dans les dérivés de polyéther soit inférieure à 0,9% en poids.

10. Procédé pour la préparation de masses de moulage dentaires selon les revendications 1 à 8, contenant des dérivés de polyéther, **caractérisé en ce que** les polyéthers oligomères cycliques sont éliminés après la fonctionnalisation des polyétherpolyols avec des groupes aziridino et/ou des groupes contenant des doubles liaisons et/ou des groupes époxyde par voie extractive, de telle manière que la teneur en polyéthers oligomères cycliques dans les dérivés de polyéther est inférieure à 0,9% en poids.

11. Procédé pour la préparation de masses de moulage dentaires selon les revendications 9 ou 10, **caractérisé en ce que** l'élimination des polyéthers oligomères cycliques est réalisée par une extraction avec des hydrocarbures en C₄ à C₁₂.

12. Utilisation de la masse de moulage dentaire selon les revendications 1 à 8 pour le moulage dans le domaine de la médecine dentaire ou de la technique dentaire.
